# EUROPÄISCHE PATENTSCHRIFT

(11) **EP 1 013 647 B1**
(45) Veröffentlichungstag und Bekanntmachung des Hinweises auf die Patenterteilung: **27.11.2002**
(21) Anmeldenummer: 99125042.4
(22) Anmeldetag: 15.12.1999
(51) Int. Cl.: C07D 239/48

(54) **Verfahren zur Herstellung von N-(Amino-4,6-dihalogenpyrimidin)-formamiden**
Process for the preparation of N-(amino-4,6-dihalogenopyrimidin)-formamides
Procédé de préparation de N-(amino-4,6-dihalogénopyrimidine)-formamides

(30) Priorität: 21.12.1998 EP 98124188; 18.01.1999 EP 99100788; 12.04.1999 EP 99107161
(43) Veröffentlichungstag der Anmeldung: 28.06.2000
(60) Teilanmeldung: 01130001.9
(73) Patentinhaber: Lonza AG, 4052 Basel (CH)
(72) Erfinder: Saikali, Elie, Dr., 19490 Worcester, PA (US); Brieden, Walter, Dr., 3902 Brig-Glis (CH)
(74) Vertreter: Ritthaler, Wolfgang, Dr.

(56) Entgegenhaltungen:
- EP-A- 0 684 236
- WO-A-91/01310
- US-A- 4 965 270

## Beschreibung

Die Erfindung betrifft ein neues Verfahren zur Herstellung von N-(Amino-4,6-dihalogenpyrimidin)formamiden der Formel 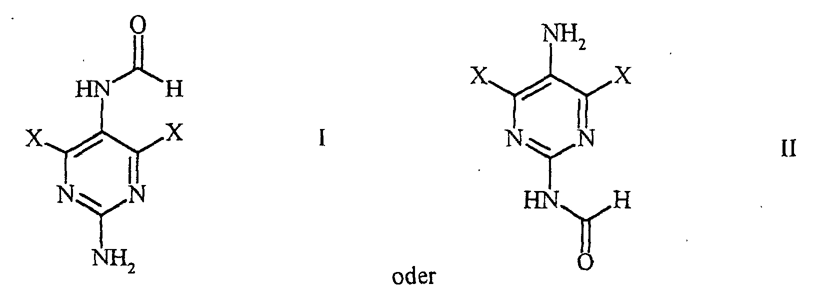 ausgehend von einem 2,5-Diamino-4,6-dihalogenpyrimidin der allgemeinen Formel

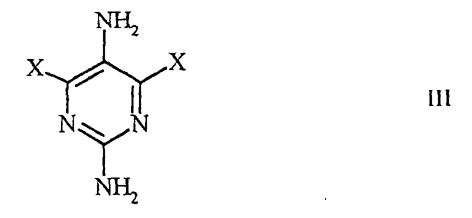

N-(Amino-4,6-dihalogenpyrimidin)formamide wie N-(2-Amino-4,6-dihalogenpyrimidin-5-yl)formamid sind wichtige Zwischenprodukte zur Herstellung von antiviralen Nukleotidderivaten (EP-A 0 684 236).

Bisher sind mehrere Verfahren zur Herstellung von N-(2-Amino-4,6-dihalogenpyrimidin-5-yl)formamid bekannt. So beschreibt bspw. die EP-A 0 684 236 ein Verfahren zur Herstellung von N-(2-Amino-4,6-dihalogenpyrimidin-5-yl)formamid ausgehend von einem Aminomalonester. Dabei wird der Aminomalonester zunächst mit Guanidin in Gegenwart eines Alkoholats zum 2,5-Diamino-4,6-dihydroxypyrimidin cyclisiert und aus diesem wird dann mit Phosphoroxychlorid in Gegenwart von Dimethylformamid das 4,6-Dichlor-N'-(dimethylaminomethylen)pyrimidin-2,5-diamin gebildet. Anschliessend wird letzteres mit wässriger Propionsäure in das gewünschte Produkt überführt. Nachteile dieses Verfahrens sind zum einen die mässige Ausbeute an gewünschtem Produkt und zum anderen, dass dieses Verfahren über 3 Verfahrensstufen läuft.

Die US-A-4 965 270 offenbart die Herstellung von 4,6-Dichlor-2,5-diformamidopyrimidin durch Umsetzung von 2,5-Diamino-4,6-dichlorpyrimidin mit Ameisensäure und Acetanhydrid.

Auch mehrere Verfahren zur Herstellung von 2,5-Diamino-4,6-dihalogenpyrimidinen wie 2,5-Diamino-4,6-dichlorpyrimidin sind bekannt. Beispielsweise beschreibt die WO 91/01310 ein Verfahren zur Herstellung von 2,5-Diamino-4,6-dichlorpyrimidin ausgehend von 2,5-Diamino-4,6-dihydroxypyrimidin in Gegenwart von Phosphoroxychlorid und einem quartären Ammoniumhalogenid oder einem schwach basischen tertiären Amin bzw. dessen Salz. Dabei dient das Phosphoroxychlorid als Lösungsmittel. Dieses Verfahren hat den Nachteil, dass es im grosstechnischen Masstab nicht reproduzierbar ist und das gewünschte Endprodukt nur in geringer Ausbeute erhalten wird.

Aufgabe der vorliegenden Erfindung war es, ein einfacheres Verfahren zur Herstellung von N-(Amino-4,6-dihalogenpyrimidin)formamiden bereit zu stellen, bei dem das gewünschte Produkt in guter Ausbeute erhalten wird.

Überraschenderweise wurde nun gefunden, dass bei der Umsetzung von 2,5-Diamino-4,6-dihalogenpyrimidin der allgemeinen Formel 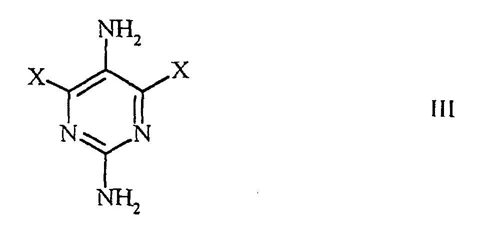 worin X ein Halogenatom bedeutet, oder von einem seiner Salze, mit Ameisensäure die Endprodukte der allgemeinen Formel I oder II direkt, d. h. ohne Zwischenstufen, in hervorragender Ausbeute erhalten werden.

Als Halogenatom können beispielsweise Cl oder Br eingesetzt werden, vorzugsweise wird Cl eingesetzt. Demzufolge wird vorzugsweise als 2,5-Diamino-4,6-dihalogenpyrimidin 2,5-Diamino-4,6-dichlor- oder 2,5-Diamino-4,6-dibrompyrimidin eingesetzt.

Geeignet für die Umsetzung sind auch die Salze der 2,5-Diamino-4,6-dihalogenpyrimidine, beispielsweise die Hydrohalogenidsalze wie die Hydrochloridsalze oder die Hydrobromidsalze.

Als Ameisensäure wird 70 - 98%ige Ameisensäure eingesetzt.

Wird die Herstellung des Produktes der Formel I gewünscht, wird eine 70-80%ige Ameisensäure eingesetzt und die Umsetzung bei einer Temperatur von 20°C bis 60°C, vorzugsweise von 25°C bis 55°C durchgeführt.

Wird die Herstellung des Produktes der Formel II gewünscht, wird eine 80-98%ige Ameisensäure eingesetzt und die Umsetzung bei einer Temperatur von 0°C bis 30°C, vorzugsweise von 10°C bis 25°C durchgeführt.

Das Edukt, das 2,5-Diamino-4,6-dihalogenpyrimidin der allgemeinen Formel III, läßt sich in guter Ausbeute erhalten, wenn man 2,5-Diamino-4,6-dihydroxypyrimidin der Formel IV 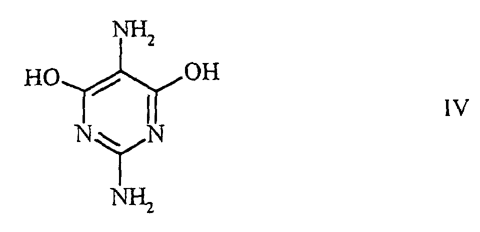 oder eines seiner Salze, in Gegenwart von einem Phosphoroxyhalogenid und einem quartären Ammoniumhalogenid oder einem Amin oder einem Salz davon mit einem halogenierten Kohlenwasserstoff als Lösungsmittel umsetzt.

Das 2,5-Diamino-4,6-dihydroxypyrimidin ist eine käufliche Verbindung. Geeignet für das Verfahren sind auch die Salze von 2,5-Diamino-4,6-dihydroxypyrimidin, beispielsweise die Hydrohalogenidsalze wie die Hydrochloridsalze bzw. Hydrobromidsalze.

Als Phosphoroxyhalogenid wird zweckmässig Phosphoroxychlorid oder Phosophoroxybromid eingesetzt.

Als Amin können primäre, sekundäre oder tertiäre Amin bzw. deren Salze, insbesondere die Hydrohalogenidsalze wie Hydrochloride oder Hydrobromide, eingesetzt werden. Geeignete Amine sind beispielsweise Mono-, Di- und Trialkylamine, wobei die Alkylreste des Amins gleich oder verschieden sein können und vorzugsweise C₁₋₆-Alkyl, insbesondere Methyl, Ethyl, Propyl oder Butyl, bedeuten. Beispiele für geeignete Amine sind Methylamin, Dimethylamin Trimethylamin, Ethylamin, Diethylamin, Triethylamin und Methylethylamin, sowie deren Hydrochloride oder Hydrobromide.

Geeignete quartäre Ammoniumhalogenide sind beispielsweise Tetraalkylammoniumhalogenide, worin die Alkylreste gleich oder verschieden sein können und vorzugsweise die oben genannte Bedeutung haben. Zu nennen sind hier beispielsweise Tetramethylammoniumhalogenide oder Tetraethylammoniumhalogenide. Zweckmäßig werden als Halogenide der quartären Ammoniumverbindungen die Chloride oder Bromide eingesetzt.

Üblicherweise wird das Amin bzw. das quartäre Ammoniumhalogenid im Überschuss, bezogen auf das 2,5-Diamino-4,6-dihydroxypyrimidin, eingesetzt; vorzugsweise werden 1 bis 10 mol Amin, ggf. in Form des Salzes oder des quartären Ammoniumhalogenids, pro 1 mol 2,5-Diamino-4,6-dihydroxypyrimdin eingesetzt.

Zweckmässig wird die Umsetzung bei einer Temperatur von 20°C bis zur Rückflusstemperatur des entsprechenden Lösungsmittels, vorzugsweise von 100 bis 120°C, durchgeführt.

Als halogenierte Kohlenwasserstoffe können halogenierte aliphatische Kohlenwasserstoffe verwendet werden. Beispiele für halogenierte aliphatische Kohlenwasserstoffe sind halogenierte Alkane. Als halogeniertes Alkan kann halogeniertes Propan wie 1,2,3-Trichlorpropan eingesetzt werden.

### Beispiele

### Beispiel 1

### Herstellung von 2,5-Diamino-4,6-dichlorpyrimidin

2,5-Diamino-4,6-dihydroxypyrimidinhydrochlorid (0,14 mol, 25 g) wurde in einen trockenen Reaktor gefüllt. Dann wurde trockenes 1,2,3-Trichlorpropan (51,96 ml) hinzugefügt und das Ganze gerührt. Anschliessend wurden Tetramethylammoniumchlorid (0,29 mol, 31,25 g) und dann POCl₃ (0,54 - 0,81 mol, 124,9 - 83,28 g, 50,6 - 75,9 ml) hinzugegeben. Die Reaktion wurde auf Rückflusstemperatur (ca. 115 °C) 24 h lang erhitzt. Dann wurde die Reaktion auf unter 50 °C abgekühlt, Eiswasser (24,44 mol, 440,44 g) hinzugefügt und das Ganze unter 55°C gehalten. Anschliessend wurde die Reaktion auf einen pH-Wert zwischen 6,5 und 7,0 mit 50%iger NaOH (3,12 mol, 124,92 g, 163,3 ml) eingestellt und die Temperatur unter 55 °C gehalten. Die Reaktion wurde 30 min. zwischen 50 und 60 °C gerührt. Dann wurde Tetrahydrofuran (3,7 mol, 267,0 g, 300 ml) zugegeben. Um ungewünschtes Material zu entfernen, wurde das Ganze über Celite abfiltriert und dann der Filterkuchen mit Essigsäureethylester (20,5 mol, 1806,58 g, 2002,86 ml) für die anschliessende Extraktion gewaschen. Die organische Phase (Tetrahydrofuran und Essigsäureethylester) wurde 3mal mit Wasser (5,57 mol, 100,32 g, 100,32 1) gewaschen, über NaHCO₃ getrocknet und filtriert. Essigsäureethylester wurde durch Vakuumdestillation entfernt. Dann wurde Hexan (0,77 mol. 66,14 g, 100,36 ml) zum restlichen organischen Material hinzugegeben, das Ganze auf unter 10 °C abgekühlt, filtriert und dann im Vakuum bei 50 °C getrocknet. Das Titelprodukt (0,09 mol, 15,71 g) wurde als leicht bräunlicher Feststoff erhalten, entsprechend einer Ausbeute von ca. 65 %, bez. auf eingesetztes 2,5-Diamino-4,6-dihydroxypyrimidin.

### Beispiel 2

### Herstellung von N-(2-Amino-4,6-dichlorpyrimidin-5-yl)formamid

2,5-Diamino-4,6-dichlorpyrimidin (0,01 mol; 2,0 g) und Wasser (0,25 mol; 4,55 ml) wurden bei Raumtemperatur gerührt. Dann wurde 98%ige Ameisensäure (0,4 mol; 18,27 g; 14,97 ml) zur Reaktion hinzugegeben. Anschliessend wurde die Reaktion auf 50 - 55 °C erwärmt und 3 h bei dieser Temperatur gehalten.
Dann wurde Toluol (0,38 mol; 34,6 g; 40 ml) für die azeotrope Destillation unter hohem Vakuum bei 50 °C hinzugegeben (Toluol wurde, um eine gute Destillation zu gewährleisten, 2mal hinzugegeben, also insgesamt 80 ml).
Anschliessend wurde das Produkt filtriert, mit Wasser gewaschen und dann unter Vakuum bei 60 °C getrocknet. Es wurden 0,01 mol (2,0 g) des obengenannten Produktes, entsprechend einer Ausbeute von ca. 90%, erhalten.

### Beispiel 3

### Herstellung von N-(5-Amino-4,6-dichlorpyrimidin-2-yl)formamid

2,5-Diamino-4,6-dichlorpyrimidin (0,001 mol; 2,0 g) und 98%ige Ameisensäure (0,5 mol, 22,96 g, 18,8 ml) wurden bei Raumtemperatur über Nacht gerührt. Dann wurde Toluol (0,94 mol, 86,76 g, 18,82 ml) hinzugegeben und die Reaktion wurde auf 0 - 5°C abgekühlt.
Das Produkt wurde abfiltriert und mit Wasser (1,11 mol, 20,0 g, 20,0 ml) gewaschen. Anschliessend wurde das Produkt unter Vakuum bei 50°C getrocknet. N-(5-Amino-4,6-dichlorpyrimidin-2-yl)formamid wurde im ¹H-NMR als einziges Produkt nachgewiesen.
Es wurden 0,01 mol (1,62 g) des obengenannten Produktes, entsprechend einer Ausbeute von ca. 70%, erhalten.

## Patentansprüche

1. Verfahren zur Herstellung von N-(Amino-4,6-dihalogenpyrimidin)formamiden der Formel 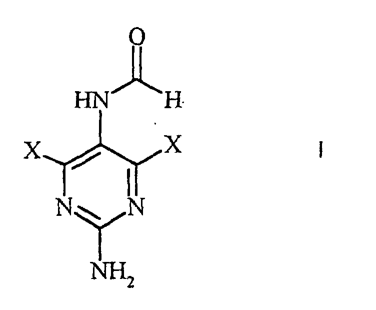 worin X ein Halogenatom bedeutet, umfassend die Umsetzung eines 2,5-Diamino-4,6-dihalogenpyrimidins der allgemeinen Formel 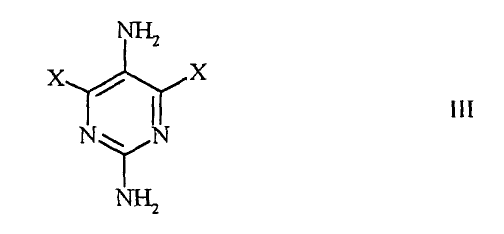 worin X die genannte Bedeutung hat, oder eines seiner Salze, mit einer 70 bis 80%igen Ameisensäure bei einer Temperatur von 20 bis 60 °C zum Endprodukt gemäss Formel I.

2. Verfahren zur Herstellung von N-(Amino-4,6-dihalogenpyrimidin)formamiden der Formel 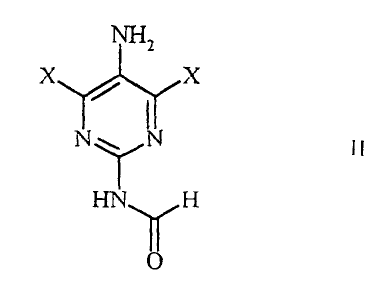 worin X ein Halogenatom bedeutet, umfassend die Umsetzung eines 2,5-Diamino-4,6-dihalogenpyrimidins der allgemeinen Formel 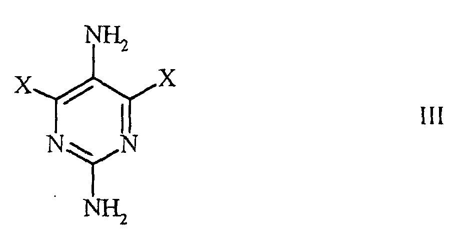 worin X die genannte Bedeutung hat, oder eines seiner Salze, mit einer 80 bis 98%igen Ameisensäure bei einer Temperatur von 0 bis 30 °C zum Endprodukt gemäss Formel II.

3. Verfahren nach Anspruch 1 oder 2, **dadurch gekennzeichnet, dass** das 2,5-Diamino-4,6-dihalogenpyrimidin der allgemeinen Formel 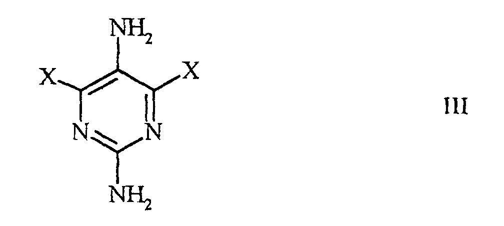 derart hergestellt wird, dass man ein 2,5-Diamino-4,6-dihydroxypyrimidin der Formel 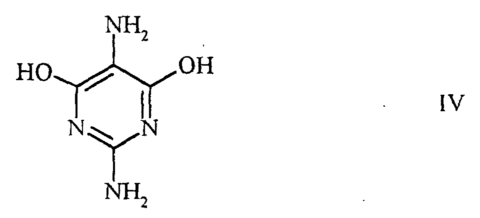 oder eines seiner Salze, mit einem Phosphoroxyhalogenid und einem quartären Ammoniumhalogenid oder einem Amin oder einem Salz davon in einem halogenierten Kohlenwasserstoff als Lösungsmittel umsetzt.

4. Verfahren nach Anspruch 3, **dadurch gekennzeichnet, dass** man die Umsetzung bei einer Temperatur von 20 °C bis zur Rückflusstemperatur des entsprechenden Lösungsmittels durchführt.

5. Verfahren nach einem der Ansprüche 3 oder 4, **dadurch gekennzeichnet, dass** man als Lösungsmittel ein halogeniertes Alkan einsetzt.

## Claims

1. Process for the preparation of N-(amino-4,6-dihalopyrimidine)formamides of the formula 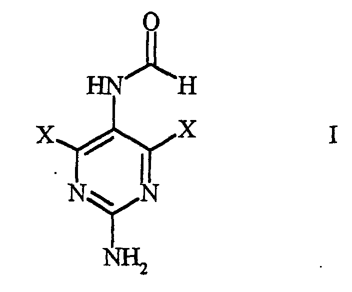 in which X is a halogen atom, comprising the reaction of a 2,5-diamino-4,6-dihalopyrimidine of the general formula 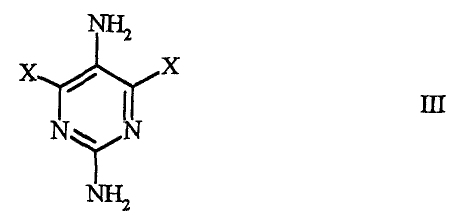 in which X has the meaning mentioned, or of one of its salts with a 70 to 80% strength formic acid at a temperature of 20 to 60°C to give the final product according to formula I.

2. Process for the preparation of N-(amino-4,6-dihalopyrimidine)formamides of the formula 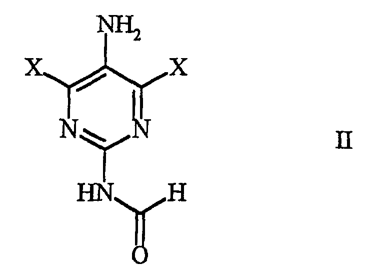 in which X is a halogen atom, comprising the reaction of a 2,5-diamino-4,6-dihalopyrimidine of the general formula 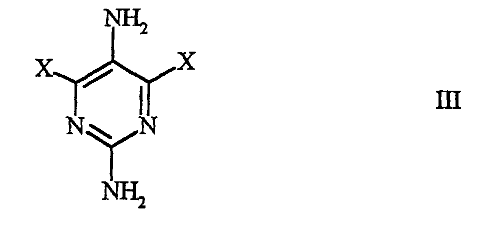 in which X has the meaning mentioned, or of one of its salts with an 80 to 98% strength formic acid at a temperature of 0 to 30°C to give the final product according to formula II.

3. Process according to Claim 1 or 2, **characterized in that** the 2,5-diamino-4,6-dihalopyrimidine of the general formula 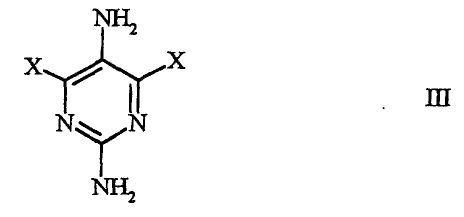 is prepared by reacting a 2,5-diamino-4,6-dihydroxypyrimidine of the formula 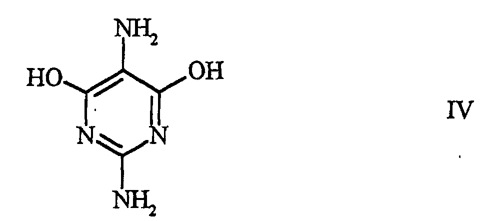 or one of its salts with a phosphorus oxyhalide and a quaternary ammonium halide or an amine or a salt thereof in a halogenated hydrocarbon as a solvent.

4. Process according to Claim 3, **characterized in that** the reaction is carried out at a temperature of 20°C up to the reflux temperature of the appropriate solvent.

5. Process according to one of Claims 3 or 4, **characterized in that** the solvent employed is a halogenated alkane.

## Revendications

1. Procédé pour la préparation de N-(amino-4,6-dihalogénopyrimidine)formamides de la formule 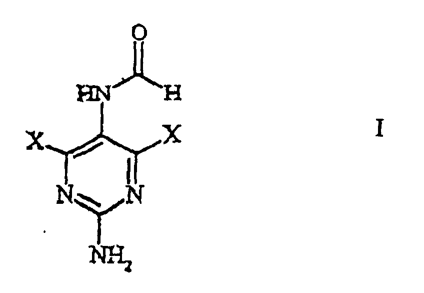 dans laquelle X signifie un atome d'halogène, comprenant la réaction d'une 2,5-diamino-4,6-dihalogénopyrimidine de la formule générale 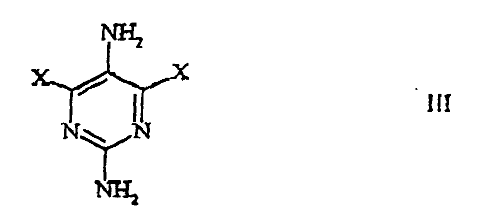 dans laquelle X a la signification citée, ou un de ses sels, avec un acide formique à 70 jusqu'à 80% à une température de 20 à 60 °C pour obtenir le produit final selon la formule I.

2. Procédé pour la préparation de N-(amino-4,6-dihalogénopyrimidine)formamides de la formule 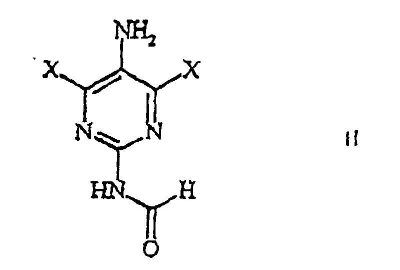 dans laquelle X signifie un atome d'halogène, comprenant la réaction d'une 2,5-diamino-dihalogénopyrimidine de la formule générale 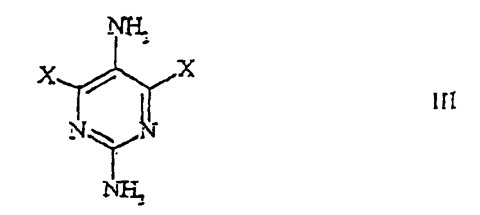 dans laquelle X a la signification citée, ou un de ses sels, avec un acide formique à 80 jusqu'à 98% à une température de 0 à 30°C pour obtenir le produit final selon la formule II.

3. Procédé selon la revendication 1 ou 2, **caractérisé en ce que** la 2,5-diamino-4,6-dialogénopyrimidine de la formule générale 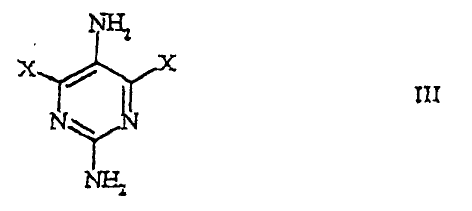 est préparée de telle sorte que l'on met en réaction une 2,5-diamino-4,6-dihydroxypyrimidine de la formule 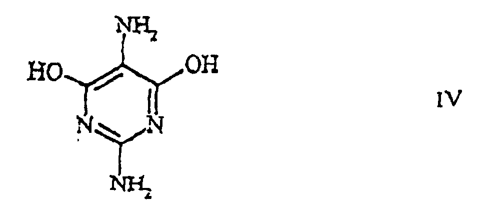 ou un de ses sels, avec un oxyhalogénure de phosphore ou un halogénure d'ammonium quaternaire ou une amine ou son sel dans un hydrocarbure halogéné en tant que solvant.

4. Procédé selon la revendication 3, **caractérisé en ce que** l'on conduit la réaction à une température de 20°C jusqu'à la température de reflux du solvant correspondant.

5. Procédé selon l'une des revendications 3 ou 4, **caractérisé en ce qu'**en tant que solvant on utilise un alcane halogéné.
